# EUROPEAN PATENT APPLICATION

(11) **EP 3 786 282 A1**
(43) Date of publication of application: **03.03.2021**
(21) Application number: 19792143.0
(22) Date of filing: 02.04.2019
(51) Int. Cl.: C12N 1/04

(54) **CRYOPRESERVATION SOLUTION AND CRYOPRESERVATION METHOD**

(30) Priority: 26.04.2018 JP 2018084679
(71) Applicant: Mitsubishi Paper Mills Limited, Tokyo 130-0026 (JP)
(72) Inventor: MATSUZAWA, Atsushi, Tokyo 130-0026 (JP)
(74) Representative: Cabinet Beau de Loménie
(86) International application number: PCT/JP2019/014589
(87) International publication number: WO 2019/208120

(57) **Abstract**

The present invention relates to a cryopreservation solution containing polyvinyl alcohol having a saponification degree of 84 mol% or lower.

## Description

### TECHNICAL FIELD

The present invention relates to a cryopreservation solution used for cryopreservation of cells or tissues, and a cryopreservation method that uses the cryopreservation solution.

### BACKGROUND ART

Excellent preservation techniques for cells or tissues of living organisms are desired in various industrial fields. In general, cells or tissues harvested from living bodies gradually become inactive even in a culture medium, and hence long-term culture of cells or tissues in vitro is undesirable. For this reason, techniques for long-term preservation of cells or tissues without the loss of biological activity are essential. Excellent preservation techniques allow more accurate analysis of cells or tissues harvested. Such excellent preservation techniques also allow transplantation of cells or tissues with their biological activity kept at a higher level, thus likely resulting in an improvement in the engraftment rate. Such excellent techniques also allow in-advance production and preservation of artificial tissues for transplantation, such as skins cultured in vitro and cell sheets formed in vitro, and storage thereof until needed. Therefore, such excellent preservation techniques are expected to bring great advantages not only in the research and medical science fields but also in the industrial field.

One of known methods for preserving cells or tissues is a slow freezing method, for example. In this method, cells or tissues are immersed in a cryopreservation solution prepared by adding a cryoprotectant to a physiological solution such as phosphate buffered saline. Examples of the cryoprotectant include compounds such as glycerol and ethylene glycol. The cells or tissues immersed in the cryopreservation solution are cooled down to -30°C to -35°C at a relatively slow cooling rate (for example, 0.3°C to 0.5°C/min), and thereby the cryopreservation solution inside and outside the cells or tissues are sufficiently cooled and become viscous. Further cooling down the cells or tissues in such a state in the preservation solution to the temperature of liquid nitrogen (-196°C) causes a slight amount of the solution both inside and surrounding the cells or tissues to solidify while the amorphous state thereof is maintained, that is, to vitrify. The vitrification (i.e., solidification) of the solution inside and outside the cells or tissues substantially immobilizes the molecules. Thus, the vitrified cells or tissues can be semipermanently preserved in liquid nitrogen.

However, since the slow freezing method requires cooling at a relatively slow cooling rate, the procedure of cryopreservation takes a long time. Further, this technique disadvantageously requires a device or jig for controlling the cooling rate. In addition, the slow freezing method cannot avoid formation of ice crystals in the preservation solution outside the cells or tissues, which may cause physical damage to the cells or tissues.

One proposed solution to the problems of the slow freezing method is a vitrification cryopreservation method. The vitrification cryopreservation method is a technique based on the principle that addition of a large amount of a cryoprotectant, such as glycerol, ethylene glycol, or dimethyl sulfoxide (DMSO), to a cryopreservation solution decreases the freezing point of the preservation solution, thereby restraining formation of ice crystals at sub-zero temperatures. When quickly cooled in liquid nitrogen, the cryopreservation solution can solidify without formation of ice crystals. This solidification is called vitrification.

The specific procedure of the vitrification cryopreservation method includes immersing cells in a cryopreservation solution and cooling the cells at the temperature of liquid nitrogen (-196°C). Since the vitrification cryopreservation method is such a simple and quick process, it advantageously does not require a long-term procedure of cryopreservation or the use of any temperature-controlling device or jig.

The vitrification method does not cause formation of ice crystals either inside or outside the cells, and thus can avoid physical damage (freezing damage) to the cells during freezing and thawing. However, successful vitrification requires a highly concentrated cryoprotectant in a preservation solution for vitrification. Yet, a highly concentrated cryoprotectant in a cryopreservation solution is highly chemically toxic to the cells. Thus, preferably, the concentration of the cryoprotectant is reduced as much as possible in order to reduce cytotoxicity. Vitrification cryopreservation with a cryopreservation solution containing a cryoprotectant at a low concentration is known to require a higher freezing rate.

In view of increasing the freezing rate of cells or tissues, a smaller amount of the cryopreservation solution around cells or tissues is better during cryopreservation of the cells or tissues. The smaller the amount of the cryopreservation solution present around cells or tissues, the lower the heat capacity of the object to be frozen and the higher the freezing rate of the cells or tissues, which is preferred for vitrification. Further, a smaller amount of the cryopreservation solution present around cells or tissues is also preferred because the cryopreservation solution is quickly diluted in a thawing solution during thawing of the frozen cells or tissues, and re-formation of ice crystals in the cells or tissues can be inhibited. Still further, the concentration of a cryoprotectant that gets mixed with a thawing solution during thawing can be reduced, which can thus advantageously reduce chemical toxicity derived from the cryoprotectant.

Various examples of cryopreservation of cells or tissues by the vitrification cryopreservation method using various processes and various cells or tissues have been reported. For example, Patent Literature 1 shows high usefulness of application of the vitrification cryopreservation method to animal or human reproductive or somatic cells in terms of viability after cryopreservation and thawing, and describes a cryopreservation solution containing 5.5 M ethylene glycol and 1 M sucrose, and a cryopreservation solution containing 40 mass% ethylene glycol and 0.3 M trehalose, for example.

The vitrification cryopreservation method is a technique which has been developed mainly using human reproductive cells. More recently, its application to iPS or ES cells has also been widely examined. Non-Patent Literature 1 discloses the effectiveness of the vitrification cryopreservation method in preservation of Drosophila embryos. Patent Literature 2 discloses the effectiveness of the vitrification cryopreservation method in preservation of plant culture cells and tissues. The former describes a cryopreservation solution containing ethylene glycol, glycol, propylene glycol, glycerol, and DMSO as cryoprotectants. The latter describes examples of cryoprotectants (freeze protection agents) in a cryopreservation solution, such as DMSO, propylene glycol, glycerol, polyethylene glycol, butanediol, formamide, propanediol, sorbitol, and mannitol. As described, the vitrification cryopreservation method is known to be useful for preservation of a wide range and different kinds of cells and tissues.

Patent Literature 3 proposes a cryopreservation method with excellent viability, which includes depositing eggs or embryos with a cryopreservation solution on a material for removing a preservation solution, and removing an excess cryopreservation solution surrounding the eggs or embryos by downward suction. The cryopreservation solution contains ethylene glycol, glycerol, and sucrose.

Patent Literature 4 suggests a cryopreservation solution containing 1.5% polyvinyl alcohol as a cryopreservation solution of cells or tissues for the slow freezing method, mainly in order to protect the cells, i.e., to achieve high viability. Patent Literature 5 and Patent Literature 6 each describe polyvinyl alcohol as a cell protectant or cryoprotectant in a cryopreservation solution for the vitrification cryopreservation method.

According to Patent Literature 7, a problem of not being able to efficiently culture cells on a hydrophobic culture substrate surface was ameliorated by a cell culture medium containing a water-soluble synthetic polymer. Patent Literature 7 describes polyvinyl alcohol as an example of the synthetic polymer. According to Patent Literature 8, cells or tissues can be easily released and recovered during thawing with the use of a device for cryopreservation which includes a layer containing a water-soluble polymer compound on an outermost surface on which the cells or tissues are to be deposited.

### CITATION LIST

### - Patent Literature

Patent Literature 1: JP 3044323 B
Patent Literature 2: JP 2008-5846 A
Patent Literature 3: WO 2011/070973
Patent Literature 4: JP 2005-261413 A
Patent Literature 5: JP 2010-213692 A
Patent Literature 6: JP 2013-111017 A
Patent Literature 7: JP 2007-124982 A
Patent Literature 8: JP 2017-60457 A

### - Non-Patent Literature

Non-Patent Literature 1: Steponkus et al., Nature 345: 170-172 (1990)

### SUMMARY OF INVENTION

### - Technical problem

As described above, in vitrification cryopreservation, cells or tissues are deposited with a small amount of a cryopreservation solution, thus achieving high cell or tissue viability. However, when cells or tissues are deposited and frozen with a small amount of a cryopreservation solution, the cells or tissues such as eggs or embryos on a sheet often adhere to a surface of a deposition part during thawing after freezing, depending on the types and conditions of the cells or tissues, for example, and recovering them thus disadvantageously requires high-level skills. Further, as a worker cryopreserves cells or tissues with a smaller amount of a cryopreservation solution, which is a preferred condition for freezing, the cells or tissues disadvantageously exhibit stronger adhesion to the surface of the deposition part during thawing after freezing.

In particular, in Patent Literature 3, a material for removing a vitrification preservation solution is used on a portion on which embryos or eggs are deposited so as to remove an excess cryopreservation solution, without the need for a worker to remove the excess cryopreservation solution surrounding the eggs or embryos, and to freeze the eggs or embryos with a small amount of the cryopreservation solution. In this regard, this technique provides good working efficiency. However, in rare cases, absorption of the preservation solution by a preservation solution absorber causes particularly strong adhesion of the eggs or embryos to the preservation solution absorber. Thus, recovering these eggs or embryos requires high-level skills in some cases.

A main object of the present invention is to provide a cryopreservation solution of a cell or tissue and a cryopreservation method which allow easy and reliable cryopreservation of a cell or tissue. Specifically, the present invention aims to provide a cryopreservation solution and a cryopreservation method which provide good working efficiency in pipetting during freezing and which allow easy recovery of a cell or tissue without causing the cell or tissue to adhere to a surface of a deposition part during thawing.

### - Solution to problem

As a result of extensive studies to solve the above problems, the present inventor found that the above problems can be solved by the following means:

(1) a cryopreservation solution containing polyvinyl alcohol having a saponification degree of 84 mol% or lower;
(2) the cryopreservation solution according to (1) above, wherein the polyvinyl alcohol has a saponification degree of 76 mol% or lower; and
(3) a cryopreservation method including immersing an equilibrated cell or tissue in the cryopreservation solution according to (1) or (2) above; and vitrifying the cell or tissue using a cryogenic coolant.

### - Advantageous Effects of Invention

The invention according to (1) above can provide a cryopreservation solution which provides good working efficiency in pipetting during freezing for vitrification cryopreservation of a cell or tissue, and which allows easy recovery of the cell or tissue without causing the cell or tissue to adhere a surface of a deposition part during thawing.

The invention according to (2) above can provide a cryopreservation solution which provides particularly excellent recoverability of a cell or tissue without causing the cell or tissue to adhere to a surface of a deposition part during thawing.

The invention according to (3) above can provide a cryopreservation method which provides good working efficiency in pipetting during freezing for vitrification cryopreservation of a cell or tissue, and which allows easy recovery of the cell or tissue without causing the cell or tissue to adhere to a surface of a deposition part during thawing.

### DESCRIPTION OF EMBODIMENTS

The cryopreservation solution of the present invention is used for cryopreservation of cells or tissues. In the present invention, the "cell" encompasses not only a single cell but also a biological cell population composed of multiple cells. The "cell population composed of multiple cells" may be a colony or cluster composed of a single kind of cells or may be a colony or cluster composed of multiple kinds of cells. The "tissue" may be composed of a single kind of cells or may be composed of multiple kinds of cells, or may contain a non-cellular substance like an extracellular matrix in addition to cells.

The cryopreservation solution of the present invention is used for cryopreservation, preferably for vitrification cryopreservation. Specifically, the cryopreservation solution of the present invention can be suitably used for cryopreservation of embryos or eggs in the Cryotop method and other methods.

Freezing in the Cryotop method is generally performed by the following steps. Cells or tissues such as embryos or eggs are immersed in an equilibration solution. Subsequently, using a thin tubular device such a pipette, the cells or tissues are retrieved with a small amount of the equilibration solution, and transferred into a cryopreservation solution. After the cells or tissues are immersed in a cryopreservation solution for a predetermined time, the cells or tissues are retrieved with a small amount of the cryopreservation solution, and attached dropwise with the small amount of the cryopreservation solution to a sheet of a device for cryopreservation. At this time, an excess cryopreservation solution is removed using a thin tubular device such as a pipette when the amount of the cryopreservation solution dropped is large. Subsequently, the sheet holding the cells or tissues is immersed and frozen in a coolant such as liquid nitrogen. In freezing, the cells or tissues may be pipetted with a thin tubular device in the equilibration solution or the cryopreservation solution in order to dehydrate the cells or tissues to replace the fluid in the cells or tissues with a solution containing a cryoprotectant. Next, thawing is described. The cells or tissues deposited are thawed by retrieving the cells or tissues on the sheet from the coolant and immersing them into a thawing solution. The use of the cryopreservation solution of the present invention allows easy and reliable cryopreservation of the cells or tissues because the cells or tissues on the deposition part can be easily recovered during thawing.

The "vitrification" herein refers to a method of freezing cells or tissues while inhibiting formation of ice crystals at a rapid cooling rate using a cryogenic coolant (e.g., liquid nitrogen), unlike the slow freezing method in which cells or tissues are frozen at a relatively slow cooling rate (e.g., a rate of 0.3°C to 0.5°C/min). The cooling rate in vitrification is, for example, 200°C/min or higher in the range of 0°C to -150°C as measured with a sheathed thermocouple (distal end outer diameter: 0.3 mm) available from Chino Corporation.

The cryopreservation solution of the present invention contains polyvinyl alcohol having a saponification degree of 84 mol% or lower.

Polyvinyl alcohol is known to function as a cell protectant or cryoprotectant in cryopreservation solutions. It is a known practice to add polyvinyl alcohol mainly to improve the viability after freezing and thawing. According to Patent Literature 4 described above, polyvinyl alcohol that is similarly added as a cryoprotectant functions as a substance alternative to protein to maintain the embryo cytoskeleton.

The polyvinyl alcohol in the cryopreservation solution of the present invention has a saponification degree of 84 mol% or lower, preferably 76 mol% or lower. A preferred lower limit of the saponification degree is 65 mol% or higher. Use of polyvinyl alcohol having a saponification degree in the above range allows easy recovery of cells or tissues from the deposition part during thawing for cryopreservation. Herein, the "saponification degree" means an average saponification degree of individual polyvinyl alcohol.

Preferably, the polyvinyl alcohol content of the cryopreservation solution of the present invention is 0.1 to 3 mass%. When the content is less than 0.1 mass%, releasability may be poor during thawing. When the content is more than 3 mass%, for example, handling of a thin tubular device such as a pipette may be hindered by too a high viscosity of the cryopreservation solution, and working efficiency may be poor during pipetting in the cryopreservation solution or during retrieval of cells or tissues from the cryopreservation solution. A more preferred polyvinyl alcohol content is 0.7 to 2.2 mass%.

Preferably, the cryopreservation solution of the present invention contains an organic solvent cryoprotectant. Preferred examples of the organic solvent cryoprotectant include organic solvent cryoprotectants such as ethylene glycol, DMSO, glycerol, propylene glycol, and propanediol. These organic solvent cryoprotectants may be used alone or in combination of two or more thereof. Preferably, the organic solvent cryoprotectant in the cryopreservation solution of the present invention has a concentration of 20 to 50 vol%, more preferably 25 to 45 vol%.

The cryopreservation solution of the present invention can contain a saccharide in order to adjust the osmotic pressure and to impart vitrification properties (freezing resistance) to cells or tissues. Preferred examples of the saccharide include sucrose, trehalose, glucose, raffinose, lactose, maltose, mannose, galactose, and fructose. These saccharides may be used alone or in combination of two or more thereof. Preferably, the saccharide in the vitrification cryopreservation solution of the present invention has a concentration of 0.2 to 1 M, more preferably 0.3 to 0.7 M.

The cryopreservation solution of the present invention can contain a polymer compound in order to adjust the viscosity and to obtain a cell protection effect. Examples of the polymer compound include polymer compounds such as polyethylene glycol, Ficoll (a copolymer of sucrose and epichlorohydrin), dextran, polyvinylpyrrolidone, and albumin; and thickening polysaccharides such as hyaluronic acid, gellan gum, xanthan gum, carrageenan, alginic acid derivatives and their salts such as sodium alginate, and cellulose derivatives and their salts such as methyl cellulose, ethyl cellulose, hydroxyethyl cellulose, hydroxypropyl cellulose, and carboxymethyl cellulose.

The cryopreservation solution of the present invention can contain a biological material such as serum. The components of serum have not been fully determined yet, but serum has been widely used for the cell protection effect during freezing.

In freezing using the cryopreservation solution of the present invention, the immersion time of cells or tissues in the cryopreservation solution (time from contact of cells with the cryopreservation solution to start of cooling with a coolant) depends on the composition of the cryopreservation solution to be used, but in many cases, preferably, the immersion time is within three minutes in order to avoid chemical toxicity of the cryopreservation solution. More preferably, the immersion time is within one minute and thirty seconds.

Preferably, cryopreservation using the cryopreservation solution of the present invention includes vitrifying cells using a cryogenic coolant, after equilibrating the cells or tissues and immersing the cells or tissues in the cryopreservation solution. Freezing at a rapid cooling rate may allow cryopreservation of the cells without causing formation of a large amount of ice crystals. Preferably, the coolant is liquid nitrogen.

In order to achieve freezing at a rapid cooling rate, generally, cells or tissues are deposited with a small amount of a cryopreservation solution, so that high cell or tissue viability is achieved. However, when cells or tissues are deposited and frozen with a small amount of a cryopreservation solution, the cells or tissues on a sheet often adhere to a surface of a deposition part during thawing after freezing, depending on the types and conditions of the cells or tissues, for example, and recovering the cells or tissues thus disadvantageously requires high-level skills. In particular, in the case of the method of removing an excess vitrification solution using an absorber such as filter paper as proposed in Patent Literature 3, while a high cooling rate is achieved, cells or tissues disadvantageously exhibit stronger adhesion. Yet, use of the cryopreservation solution of the present invention allows easy release of the cells or tissues during thawing, and the cryopreservation solution is thus suitably used.

The cryopreserved cells can be semipermanently preserved in a cryogenic environment where the vitrification state of the cells is maintained. The temperature at which the vitrification state is maintained varies depending on the composition of the cryopreservation solution, but in many cases, preferably, the temperature is -150°C or lower. Preferably, the environment where the above temperature is maintained is the inside of a liquid nitrogen storage container or a gas nitrogen storage container.

Freezing in the cryopreservation method of the present invention has been described above. Next, thawing is described.

Thawing of cells or tissues using the cryopreservation solution of the present invention can be performed by a commonly known method such as the Cryotop method. Cryopreserved cells or tissues deposited on a sheet are retrieved and brought into direct contact with a thawing solution whose temperature is maintained at 37°C, whereby the cryopreservation solution and the cells or tissues are thawed and the cryopreservation solution is diluted at the same time. At this time, the cells or tissues are released from the sheet in the thawing solution. Gentle release of the cells or tissues from the sheet is preferred in terms of viability after freezing and thawing. After a predetermined time, the released cells or tissues are transferred from the thawing solution into the dilute solution. The cells or tissues are immersed in the dilute solution for a predetermined time, and further transferred into a washing solution. The above procedure gradually changes the osmotic pressure to dilute and remove a cryoprotectant in the cells or tissues, whereby gentle culture conditions are restored.

Examples of the cell that can be cryopreserved using the cryopreservation solution of the present invention include reproductive cells such as eggs, embryos, and sperms from mammals (for example, human, bovine, swine, equine, leporine, rat, and mouse); and pluripotent stem cells such as induced pluripotent stem cells (iPS cells) and embryonic stem cells (ES cells). Also included are culture cells such as primary culture cells, subculture cells, and cell lines. In one or more embodiments, examples of the cell include adhesive cells such as fibroblasts, cancer-derived cells (e.g., pancreatic cancer cells and hepatoma cells), epithelial cells, vascular endothelial cells, lymphatic endothelial cells, neuronal cells, chondrocytes, tissue stem cells, and immune cells. Examples of the tissue that can be cryopreserved include tissues formed of homologous cells and tissues formed of heterologous cells, such as tissues of ovary, skin, corneal epithelium, periodontal ligament, and myocardium. The present invention is particularly suitable for cryopreservation of sheet-like tissues (e.g., cell sheets and skin tissues). The cryopreservation method of the present invention can be suitably used for cryopreservation of not only native tissues harvested from living bodies but also artificial tissues, such as cultured skins obtained by in vitro growth of cells, cell sheets formed in vitro, and a three-dimensional tissue model described in JP 2012-205516 A. The cryopreservation solution of the present invention is suitably used as a cryopreservation solution of the aforementioned cells or tissues.

### EXAMPLES

The present invention is specifically described below in further details by referring to examples, but the present invention is not limited to the following examples.

### (Example 1)

To commercially available "Medium 199" (Life Technologies) as a base solution containing L-glutamine, phenol red, and 25 mM HEPES (4-(2-hydroxyethyl)-1-piperazineethanesulfonic acid) were added ethylene glycol (15 vol%) and DMSO (15 vol%) as organic solvent cryoprotectants, sucrose (0.5 M) as a saccharide, gentamicin (50 mg/L) as an antibiotic, and polyvinyl alcohol "Kuraray Poval PVA 505" (saponification degree: 73.5 mol%) available from Kuraray Co., Ltd. (0.5 mass%). Thus, a cryopreservation solution of Example 1 was prepared.

### (Example 2)

A cryopreservation solution of Example 2 was prepared as in Example 1, except that the amount of Kuraray Poval PVA 505 was changed from 0.5 mass% to 0.9 mass%.

### (Example 3)

A cryopreservation solution of Example 3 was prepared as in Example 1, except that the amount of Kuraray Poval PVA 505 was changed from 0.5 mass% to 1.8 mass%.

### (Example 4)

A cryopreservation solution of Example 4 was prepared as in Example 1, except that the amount of Kuraray Poval PVA 505 was changed from 0.5 mass% to 2.5 mass%.

### (Example 5)

A cryopreservation solution of Example 5 was prepared as in Example 1, except that Kuraray Poval PVA 505 was not added and that polyvinyl alcohol "Kuraray Poval PVA 403" (saponification degree: 80 mol%) available from Kuraray Co., Ltd. (0.9 mass%) was added.

### (Comparative Example 1)

A cryopreservation solution of Comparative Example 1 was prepared as in Example 1, except that polyvinyl alcohol "Kuraray Poval PVA 505" was not added.

### (Comparative Example 2)

A cryopreservation solution of Comparative Example 2 was prepared as in Example 1, except that Kuraray Poval PVA 505 was not added and that polyvinyl alcohol "Gohsenol® EG-05P" (saponification degree: 88 mol%) available from The Nippon Synthetic Chemical Industry Co., Ltd. (0.9 mass%) was added.

### (Comparative Example 3)

A cryopreservation solution of Comparative Example 3 was prepared as in Example 1, except that Kuraray Poval PVA 505 was not added and that polyvinyl alcohol "Kuraray Poval PVA 617" (saponification degree: 95 mol%) available from Kuraray Co., Ltd. (0.9 mass%) was added.

### <Preparation of sphere>

A sphere for use in cell or tissue releasability evaluation was prepared as follows. Mouse embryonic fibroblasts (MEF cells) were cultured on a cell culture petri dish, and the fibroblasts were released and recovered by trypsin. Subsequently, the fibroblasts were seeded on a PrimeSurface 96U plate available from Sumitomo Bakelite Co., Ltd. at a cell concentration of 50 cells/well, and subjected to suspension culture, whereby sphere formation was induced. After culturing for three days, a sphere having a diameter of about 100 µm was obtained.

### <Preparation of device 1 for cryopreservation>

A device for cryopreservation to be used for cryopreservation of cells or tissues was prepared as follows. Hot melt urethane resin "Purmelt® QR 170-7141P" available from Henkel Japan Ltd. was applied to a dry solids content of 30 g/m² to form an adhesive layer on a transparent PET film. The adhesive layer was not applied to a deposition part region but only to the periphery of the region. Before the adhesive layer was fully cured, hydrophilized porous polytetrafluoroethylene (pore size: 0.2 µm; porosity: 71%; thickness: 35 µm) available from Advantec Toyo Kaisha, Ltd. as a preservation solution absorber was bonded to the adhesive layer, whereby a deposition part on which cells or tissues were to be deposited was obtained. The deposition part was cut to a size of 1.5 mm × 20 mm, and a short side of the deposition part was bonded to a stick-shaped ABS resin handle, whereby a device 1 for cryopreservation was produced.

### <Freezing>

The sphere prepared as described above was recovered from the culture medium, and transferred into an equilibration solution with a small amount of the culture medium using a thin tubular device called a stripper pipetter (hereinafter, pipette). The composition of the equilibration solution contained Medium 199 described above as a base solution, and 7.5 vol% ethylene glycol, 7.5 vol% DMSO, and 50 mg/L gentamicin. The sphere was immersed in the equilibration solution for three minutes, and then transferred into each of the cryopreservation solutions of Examples 1 to 5 and Comparative Examples 1 to 3 with a small amount of the equilibration solution using a pipette. The sphere was pipetted several times in the cryopreservation solution. Subsequently, at one minute after immersion in the cryopreservation solution, the sphere was attached dropwise with a very small amount of the cryopreservation solution to the deposition part of the device 1 for cryopreservation using a pipette. After the sphere was attached dropwise, microscopic observation was made for automatic absorption of the cryopreservation solution into the preservation solution absorber. After the observation showed that an excess vitrification solution was mostly absorbed, the sphere on the deposition part of the device 1 for cryopreservation was immersed and frozen in liquid nitrogen. Thus, freezing was performed. The frozen device 1 for cryopreservation was stored in the liquid nitrogen until thawing.

### <Evaluation of working efficiency in freezing>

The working efficiency during pipetting in the cryopreservation solution in freezing was evaluated based on the following criteria. The results are shown in "Evaluation of working efficiency in freezing" in Table 1.

The working efficiency in freezing was evaluated based on the following criteria.
Good: The cryopreservation solution had liquid properties that allowed easy pipetting, and the working efficiency of pipetting and the like in the cryopreservation solution was good.
Fair: The cryopreservation solution had liquid properties that slightly interfered with pipetting, but a series of procedures such as pipetting in the cryopreservation solution was feasible.
Poor: The cryopreservation solution had liquid properties that interfered with pipetting, making pipetting difficult.

### <Thawing>

The sphere frozen using the cryopreservation solution of each of Examples 1 to 5 and Comparative Examples 1 to 3 was thawed by the following procedure. The device for cryopreservation with the frozen sphere was retrieved from the liquid nitrogen, and the deposition part of the device for cryopreservation holding the sphere was immersed in a thawing solution whose temperature was maintained at 37°C. The composition of the thawing solution contained Medium 199 described above as a base solution, 1 M sucrose, and 50 mg/L gentamicin. While microscopic observation was made for the deposition part immersed in the thawing solution, attempts were made to release the sphere from the deposition part. The sphere was released as follows. After the deposition part was immersed in the thawing solution, release of the sphere from the deposition part was simply observed for 60 seconds. When the sphere was not released after 60 seconds of immersion in the thawing solution, the deposition part was shaken in an attempt to release the sphere.

### <Evaluation of releasability in thawing>

The releasability of the sphere from the deposition part in thawing was evaluated based on the following criteria. The results are shown in "Evaluation of releasability in thawing" in Table 1.

The releasability in thawing was evaluated based on the following criteria.
Excellent: The sphere was released from the deposition part within 60 seconds of immersion in the thawing solution. Good: The sphere was released from the deposition part by shaking the deposition part after 60 seconds of immersion in the thawing solution.
Poor: The sphere adhered to a degree that prevented easy recovery of the sphere, and the sphere could not be released, or the cells making up the sphere were disaggregated during recovery.

**[Table 1]**

| | Saponification degree of PVA [mol%] | PVA content [wt%] | Evaluation of working efficiency in freezing | Evaluation of releasability in thawing |
|---|---|---|---|---|
| Example 1 | 73.5 | 0.5 | Good | Good |
| Example 2 | 73.5 | 0.9 | Good | Excellent |
| Example 3 | 73.5 | 1.8 | Good | Excellent |
| Example 4 | 73.5 | 2.5 | Fair | Excellent |
| Example 5 | 80 | 0.9 | Good | Good |
| Comparative Example 1 | - | 0 | Good | Poor |
| Comparative Example 2 | 88 | 0.9 | Good | Poor |
| Comparative Example 3 | 95 | 0.9 | Fair | Poor |

The results in Table 1 show that the cryopreservation solutions of the present invention have excellent working efficiency during freezing and excellent releasability during thawing.

### (Comparative Example 4)

A 5 mass% aqueous solution of polyvinyl alcohol "Kuraray Poval PVA 505" (saponification degree: 73.5 mol%) available from Kuraray Co., Ltd. was applied to a dry solids content of 5 g/m² by slide hopper coating to the preservation solution absorber (hydrophilized porous polytetrafluoroethylene) of the device 1 for cryopreservation produced as described above. The resulting coating was dried at room temperature, and heated at 120°C for 40 hours. Thus, a device 2 for cryopreservation was obtained.

### <Freezing>

In the freezing described above, the sphere was immersed in the equilibration solution for three minutes, and then transferred with a small amount of the equilibration solution into the cryopreservation solution of Comparative Example 1 using a pipette. The sphere was pipetted several times in the cryopreservation solution. Subsequently, at one minute after immersion in the cryopreservation solution, the sphere was attached dropwise with a very small amount of the cryopreservation solution to the deposition part of the device 2 for cryopreservation using a pipette. After the sphere was attached dropwise, microscopic observation was made for automatic absorption of the cryopreservation solution into the preservation solution absorber. After the observation showed that an excess vitrification solution was mostly absorbed, the sphere on the deposition part of the device 2 for cryopreservation was immersed and frozen in liquid nitrogen. The frozen device 2 for cryopreservation was stored in the liquid nitrogen until thawing.

### <Evaluation of working efficiency in freezing>

The working efficiency was evaluated based on the same criteria as those for "Evaluation of working efficiency in freezing" described above. According to the result, working efficiency equivalent to that obtained with the use of the cryopreservation solution of Comparative Example 1 was obtained.

### <Evaluation of releasability in thawing>

The working efficiency was evaluated based on the same criteria as those for "Evaluation of releasability in thawing" described above. According to the result, the sphere could not be released from the deposition part by shaking the deposition part after 60 seconds of immersion in the thawing solution, but the sphere was released from the deposition part by shaking the deposition part after 90 seconds of immersion in the thawing solution.

### INDUSTRIAL APPLICABILITY

The present invention can be applied to cryopreservation of cells or tissues for embryo transfer and artificial insemination of domestic animals (e.g., cattle) and other animals, and for human artificial insemination; iPS cells; ES cells; commonly used culture cells; cells or tissues harvested from living bodies for the purpose of examination or implantation; and cells or tissues cultured in vitro.

## Claims

1. A cryopreservation solution comprising:
polyvinyl alcohol having a saponification degree of 84 mol% or lower.

2. The cryopreservation solution according to claim 1,
wherein the polyvinyl alcohol has a saponification degree of 76 mol% or lower.

3. A cryopreservation method comprising:
immersing an equilibrated cell or tissue in the cryopreservation solution according to claim 1 or 2; and
vitrifying the cell or tissue using a cryogenic coolant.
